# EUROPÄISCHE PATENTANMELDUNG

(11) **EP 1 389 459 A1**
(43) Veröffentlichungstag der Anmeldung: **18.02.2004**
(21) Anmeldenummer: 03017712.5
(22) Anmeldetag: 02.08.2003
(51) Int. Cl.: A61K 7/48

(54) **Verwendung vo Hydroxypropylstärkephosphat in wirkstoffhaltigen kosmetischen Zubereitungen**

(30) Priorität: 16.08.2002 DE 10237459
(71) Anmelder: Beiersdorf AG, 20245 Hamburg (DE)
(72) Erfinder: Nielsen, Jens, 24558 Henstedt-Ulzburg (DE); Kröpke, Rainer, 22869 Schenefeld (DE)

(57) **Zusammenfassung**

Die vorliegende Erfindung betrifft die Verwendung von Hydroxypropylstärkephosphat (Struktur ZEA) als Hydrokolloid, allein oder in Kombination mit weiteren Hydrokolloiden zur Verbesserung der Verfügbarkeit von Wirkstoffen, die in kosmetischen und oder dermatologischen Zubereitungen enthalten sind.

## Beschreibung

Die vorliegende Erfindung betrifft die Verwendung von Hydroxypropylstärkephosphat (Struktur ZEA) als Hydrokolloid, allein oder in Kombination mit-weiteren Hydrokolloiden zur Verbesserung der Verfügbarkeit von Wirkstoffen, die in-kosmetischen und oder dermatologischen Zubereitungen enthalten sind.

Hydrokolloide werden in der kosmetischen Industrie und Nährmittelindustrie schon lange verwendet. Sie dienen als Suspendiermittel, Emulgatoren, Suspensions- und Emulsionsstabilisatoren. Der Begriff Hydrokolloide weist auf die Affinität dieser Moleküle zum Wasser hin, d.h. sie sind hydrophil. Weiterhin sind diese Moleküle Makromoleküle, die sich verschiedenen Stoffklassen zuordnen lassen. Sie können aus Pflanzen, Tieren oder Mikroorganismen isoliert werden oder synthetischen Ursprungs sein. Sie enthalten viele Hydroxylgruppen und können Polyelektrolyte sein. Sie werden kosmetische Zubereitungen oder Nährmitteln zugesetzt oder sind bereits natürlicherweise enthalten. Sie verleihen den Produkten bestimmte Eigenschaften in Bezug auf Viskosität, Wasserbindungsverhalten und Emulsionsstabilität.

In der Kosmetik finden sich Hydrokolloide in vielen galenischen Formulierungen, wie z.B. Emulsionen, Gele und viele mehr.

Hydrokolloide sind Substanzen natürlichen oder synthetischen Ursprungs. Sie bestehen aus Mischungen von ähnlichen, aber nicht identischen Molekülen. Die Zusammensetzungen werden deshalb beeinflusst von der Herkunft bzw. des Synthesewegs der Hydrokolloide und deren Aufarbeitung. Zum Verständnis der Wirkungsweise von Hydrokolloiden sind verschiedene Eigenschaften wichtig. Zum einen interagieren sie mit Wasser und reduzieren auf diese Weise das Diffusionsbestreben der Wassermoleküle. Darüber hinaus werden Wassermoleküle festgehalten. Dies kann durch Wasserstoffbrückenbindungen geschehen oder Wassermoleküle werden in interoder intramolekulare Hohlräume eingelagert.

Hydrokolloide in kosmetischen oder dermatologischen Zubereitungen beeinflussen das Fließverhalten dieser Zubereitungen. Hydrokolloide werden eingesetzt, um die Viskosität zu erhöhen. Dadurch werden folgende Phänome verhindert: Absetzen von bestimmten Inhaltsstoffen, Phasentrennungen, Kollabieren von Schaum, Kristallisationsvorgänge.

Viele verschiedene Hydrokolloide sind bekannt: Alginat, Arabinoxylan, Carrageen; Carboxymethylcellulose, Cellulose, Gelatine, β-Glucan, Guar Gummi, Pektin, Stärke, Xanthan Gummi und andere.

Gummen werden aus Pflanzen- oder Baumsäften, die an der Luft erhärten und Harze bilden oder aus Extrakten aus Wasserpflanzen gewonnen. Hierzu zählen beispielsweise: Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin werden derivatisierte Gummen wie z.B. Hydroxypropyl Guar (Jaguar® HP 8) verwendet.

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminium-silikate wie Veegum®. Diese können als solche oder in modifizierter Form verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Goodrich (Carbopol 980, 981, 1382, 5984, 2984, EDT 2001 oder Pemulen TR2).

Unter den Polymeren befinden sich z.B. Polyacrylamide (Seppigel 305), Polyvinylalkohole, PVP, PVP / VA Copolymere, Polyglycole.

Weit verbreitet ist der Einsatz von Stärke, das Reservekohlenhydrat in Pflanzenknollen und im Endosperm von Samen. Häufig genutzte Quellen sind Mais, Weizen, Kartoffeln, Tapioca und Reis. Stärke ist vielseitig einsetzbar und billig. Sie wird als Verdicker, Wasser-bindende Verbindung, Emulsionsstabilisator und Gel-bildendes Agens eingesetzt.

Stärke besteht aus Amylose (20- 30%) und Amylopektin (70-80%). Grundbaustein ist die Glucose, die in langen Ketten α-1,4-glykosidisch miteinander verknüpft sind. Im Amylopektin tritt jeweils nach etwa 20 Glucosebausteinen eine α-1,6-glykosidische Bindung auf. Dies führt zu einer Verzweigung der Zuckerketten.

Amylose besitzt wertvollere Funktionen als Hydrokolloid, deshalb ist es sinnvoll Pflanzenextrakte zu verwenden, die einen erhöhten Amyloseanteil besitzen. Die langgestreckte Konformation der Amylose bedingt eine hohe Viskosität, die darüber hinaus durch Temperaturänderungen wenig beeinflusst wird. Das Innere der Amylose ist relativ hydrophob und bindet Wassermoleküle schlecht. Bevorzugt werden Lipide und Lipid-ähnliche Verbindungen bebunden.

Neben den natürlichen, unveränderten Stärkemolekülen werden vielfach modifizierte Stärkemoleküle eingesetzt. Diese Modifizierungen haben unterschiedliche Auswirkungen auf die Eigenschaften von Stärke. So kann z. B. durch das Einfügen von Hydroxypropylgruppen die Löslichkeit der Stärkemoleküle verbessert werden.

Aus EP 912 164 B1 sind Stärke-haltige Reinigungs- und Pflegemittel bekannt, die dadurch gekennzeichnet sind, dass vorverkleistertes, hydroxypropyliertes Distärkephosphat von Wachsmaisstärke in Form lose agglomerierter Körner in verschiedenste kosmetische Zubereitungen eingearbeitet wird. Es ergeben sich für den Fachmann jedoch keine Hinweise auf Wechselwirkungen von dem vorverkleisterten, hydroxypropylierten Distärkephosphat von Wachsmaisstärke in lose agglomerierter Form und den ebenfalls in vielen Zubereitungen eingesetzten speziellen Wirkstoffen.

Auch aus US 6,248,338 B1 sind Stärke-haltige Reinigungs- und kosmetische Pflegezubereitungen bekannt, die durch einen Gehalt an vorverkleistertem, hydroxypropyliertem Distärkephosphat von Wachsmaisstärke in Form lose agglomerierter Körner gekennzeichnet sind, ebenfalls ohne dass daraus ein Hinweis für den Fachmann erkennbar wäre, ob und welche Wechselwirkungen von Stärkederivat und gegebenenfalls eingesetzten Wirkstoffen bestehen.

Aus den Produktinformationen von *National Starch and Chemical Company* zu Hydroxypropylstärkephosphat (Struktur ZEA) ist bekannt, dass diese Verbindung in kosmetische Präparate eingearbeitet werden kann und zu Verbesserungen dieser Präparate hinsichtlich der Herstellung, aber auch Optik und Sensorik führt. Auch hieraus ergeben sich für den Fachmann keine Hinweise auf Wechselwirkungen des Stärkederivates mit Wirkstoffen wie sie häufig in kosmetischen Zubereitungen eingearbeitet werden. Hydroxypropylstärkephosphat (Struktur ZEA) trägt die Handelsbezeichnung Hydroxypropylstärkephosphat auf Maisbasis, dieser Begriff wird synonym zu Structure™ ZEA und vorverkleistertes, hydroxypropyliertes Distärkephosphat von Wachsmaisstärke in Form lose agglomerierter Körner verwendet.

In heutigen kosmetischen und dermatologischen Produkten werden vielfach Wirkstoffe unterschiedlicher chemischer Natur eingearbeitet, die gegen die Hautalterung, fettige Haut, trockene Haut, Akne und viele andere Hautschädigungen oder - veränderungen wirken. Bevorzugt werden diese Wirkstoffe in Emulsionen eingearbeitet.

Der Einsatz von Hydrokolloiden, in kosmetischen oder dermatologischen Zubereitungen, insbesondere Emulsionen, führt zu einer Stabilisierung dieser Zubereitungen. Eine wichtige, oben bereits beschriebene Eigenschaft, ist die Wechselwirkung der Hydrokolloide mit Wasser und wasserlöslichen Molekülen, aber auch Lipiden und Lipid-ähnlichen Verbindungen. Hieraus ergibt sich zum einen die Notwendigkeit Hydrokolloide zur Emulsionsstabilisierung einzusetzen; zum anderen aber auch die damit verbundene Wechselwirkung dieser Hydrokolloide mit speziellen Wirkstoffen, wie z.B. Anti-Akne, Anti-Aging, Antioxidantien, Parfümen und vielen mehr. Diese Wechselwirkungen bedeuten aber auch, das die eingesetzten Wirkstoffe nicht immer in ausreichendem Maße zur Verfügung stehen.

Die Nachteile des derzeitigen Standes der Technik bestehen in der Tatsache, dass die Hydrokolloide, die in Emulsionen als Emulsionsstabilisatoren'und Verdicker eingesetzt werden, auch Wechselwirkungen mit den zugefügten Wirkstoffen eingehen, die dazu führen, dass die Wirkstoffe nicht immer in ausreichendem Maße zur Verfügung stehen und damit ihre Wirkung relativ gering, zumindest jedoch eingeschränkt ist.

Aufgabe der vorliegenden Erfindung war der Einsatz von Hydrokolloiden zur Emulsionsstabilisierung und Verdickung bei gleichzeitig ausreichender und befriedigender Verfügbarkeit von spezifischen Wirkstoffen, die in die Emulsionen eingearbeitet werden.

Überraschender Weise wurde durch die Verwendung des Hydrokolloids Hydroxypropylstärkephosphat (Struktur ZEA) als Hydrokolloid, allein oder in Kombination mit weiteren Hydrokolloiden die Verfügbarkeit von in kosmetischen und/oder dermatologischen Zubereitungen enthaltenen Wirkstoffen verbessert.

Viele Derivate der Stärke finden heute ihren Einsatz in Nahrungsmitteln und Kosmetika. Hierzu zählen vernetzte, oxidierte, acetylierte, hydroxypropylierte und teilweise hydrolysierte Stärkemoleküle. Hydroxypropylierte Stärkephosphatmoleküle werden in pulverförmigen Haarbleichmitteln und Haarfarben, in Haarspülungen, aber auch in kosmetischen Emulsionen eingesetzt. Sie dienen als Verdickungsmittel, Emulsionsstabilisierungsmittel und Sensorikverbesserer. (*A. Wendt; Parfüm und Kosmetik 79,14; 1988*)

Hyproxypropylstärkephosphat (Struktur ZEA) wird aus Mais gewonnen und hat die folgende Struktur:

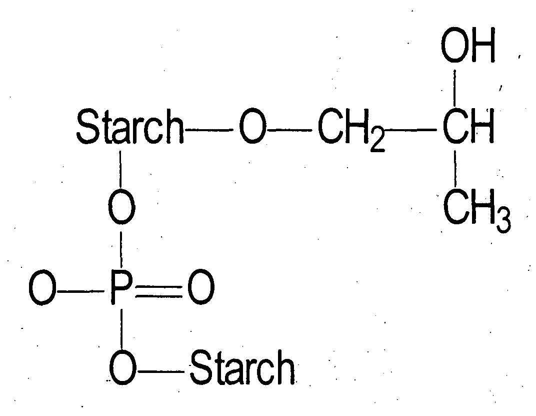

Native Stärke ist in kaltem Wasser unlöslich und kosmetische oder dermatologische Zubereitungen mit dieser Stärke rufen oft ein klebriges oder, wegen z.T. ungelöster Partikel, ein raues Hautgefühl hervor. Hydroxypropylstärkephosphat (Struktur ZEA) ist "vorverkleistert" und liegt weitgehend in Form von "agglomerierten" Stärkekörnern vor. Aufgrund dieser Vorbehandlung ist Hydroxypropylstärkephosphat (Struktur ZEA) in kaltem Wasser vollständig löslich. Eingearbeitet in kosmetische und dermatologische Zubereitungen, trägt sie zu einem angenehmen Hautgefühl bei und verbessert die Sensorik und Optik der jeweiligen Zubereitung. Hydroxypropylstärkephosphat (Struktur ZEA) ist nicht-ionisch, bevorzugt einsetzbar im alkalischen pH-Wertbereich, aber auch in einem breiteren pH-Wertbereich verwendbar. Ebenso kann Hydroxypropylstärkephosphat (Struktur ZEA) in Zubereitungen, die einen hohen Gehalt an Elektrolyten aufweisen, eingearbeitet werden und die Formulierungen bleiben stabil (aus: *Produktinformationen der Firma National Starch and Chemical Company*).

Erfindungsgemäße Emulsionen im Sinne der vorliegenden Erfindung, z.B. in Form einer Crème, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Die Lipidphase der erfindungsgemäßen kosmetischen oder dermatologischen Emulsionen kann vorteilhaft gewählt werden aus folgender Substanzgruppe:
- Mineralöle, Mineralwachse
- Öle, wie Triglyceride der Caprin- oder der Caprylsäure, ferner natürliche öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die ölphase der Emulsionen der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Solche Silicone oder Siliconöle können als Monomere vorliegen, welche in der Regel durch Strukturelemente charakterisiert sind, wie folgt:

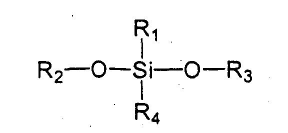

Als erfindungsgemäß vorteilhaft einzusetzenden linearen Silicone mit mehreren Siloxyleinheiten werden im allgemeinen durch Strukturelemente charakterisiert wie folgt: 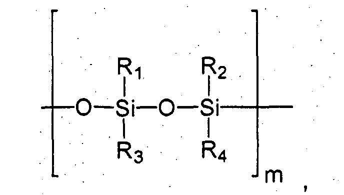 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist), m kann dabei Werte von 2 - 200.000 annehmen.

Erfindungsgemäß vorteilhaft einzusetzende cyclische Silicone werden im allgemeinen durch Strukturelemente charakterisiert, wie folgt 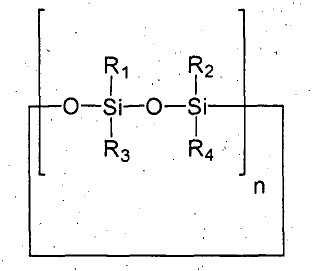 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). n kann dabei Werte von 3/2 bis 20 annehmen. Gebrochene Werte für n berücksichtigen, dass ungeradzahlige Anzahlen von Siloxylgruppen im Cyclus vorhanden sein können.

Vorteilhaft wird Cyclomethicon (z.B. Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Undecamethylcyclotrisiloxan, Polydimethylsiloxan, Poly(methylphenylsiloxan), Cetyldimethicon, Behenoxydimethicon.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-lsostearat (und) Hexyllaurat)

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate.

Erfindungsgemäße als Emulsionen vorliegenden Zubereitungen enthalten insbesondere vorteilhaft ein oder mehrere Hydrocolloide. Diese Hydrocolloide können vorteilhaft gewählt werden aus der Gruppe der Gummen, Polysaccharide, Cellulosederivate, Schichtsilikate, Polyacrylate und/oder anderen Polymeren.

Erfindungsgemäße als Emulsionen vorliegenden Zubereitungen enthalten einen oder mehrere Emulgatoren. Diese Emulgatoren können vorteilhaft gewählt werden aus der Gruppe der nichtionischen, anionischen, kationischen oder amphoteren Emulgatoren.

Unter den nichtionischen Emulgatoren befinden sich
a) Partialfettsäureester und Fettsäureester mehrwertiger Alkohole und deren ethoxylierte Derivate (z. B. Glycerylmonostearate, Sorbitanstearate, Glycerylstearylcitrate, Sucrosestearate)
b) ethoxylierte Fettalkohole und Fettsäuren
c) ethoxilierte Fettamine, Fettsäureamide, Fettsäurealkanolamide
d) Alkylphenolpolyglycolether (z.B. Triton X)

Unter den anionischen Emulgatoren befinden sich
a) Seifen (z. B. Natriumstearat)
b) Fettalkoholsulfate
c) Mono-, Di- und Trialkylphosphosäureester und deren Ethoxylate

Unter den kationischen Emulgatoren befinden sich
a) quaternäre Ammoniumverbindungen mit einem langkettigen aliphatischen Rest z.B. Distearyldimonium Chloride

Unter den amphoteren Emulgatoren befinden sich
a) Alkylamininoalkancarbonsäuren
b) Betaine, Sulfobetaine
c) Imidazolinderivate

Weiterhin gibt es natürlich vorkommende Emulgatoren, zu denen Bienenwachs, Wollwachs, Lecithin und Sterole gehören.

O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel R-COO-(-CH₂-CH₂-O-)ₙ-H,
- der veretherten Fettsäureethoxylate der allgemeinen Formel R-COO-(-CH₂-CH₂-O-)ₙ-R',
- der veresterten Fettsäureethoxylate der allgemeinen Formel R-COO-(-CH₂-CH₂-O-)ₙ -C(O)-R',
- der Polyethylenglycolglycerinfettsäureester
- der ethoxylierten Sorbitanester
- der Cholesterinethoxylate
- der ethoxylierten Triglyceride
- der Älkylethercarbonsäuren der allgemeinen Formel R-O-(-CH₂-CH₂-O-)ₙ-CH₂-COOH nd n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel R-O-(-CH₂-CH(CH₃)-O-)ₙ-H,
- der Polypropylenglycolether der allgemeinen Formel R-O-(-CH₂-CH(CH₃)-O-)ₙ-R',
- der propoxylierten Wollwachsalkohole,
- der veretherten Fettsäurepropoxylate R-COO-(-CH₂-CH(CH₃)-O-)ₙ-R',
- der veresterten Fettsäurepropoxylate der allgemeinen Formel R-COO-(-CH₂-CH(CH₃)-O-)ₙ-C(O)-R',
- der Fettsäurepropoxylate der allgemeinen Formel R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel R-O-Xₙ-Yₘ-H,
- der Polypropylenglycolether der allgemeinen Formel R-O-Xₙ-Yₘ-R',
- der veretherten Fettsäurepropoxylate der allgemeinen Formel R-COO-Xₙ-Yₘ-R',
- der Fettsäureethoxylate/propoxylate der allgemeinen Formel R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)-stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (lsosteareth-13), Polyethylenglycol(14)isostearylether (Isosteareth-14), Polyethylenglycol(15)isostearylether (lsosteareth-15), Polyethylenglycol(16)isostearylether (Isosteareth-16), Polyethylenglycol(17)isostearylether (lsosteareth-17), Polyethylenglycol(18)isostearylether (lsosteareth-18), Polyethylenglycol(19)isostearylether (Isosteareth-19), Polyethylenglycol(20)isostearylether (lsosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (lsoceteth-13), Polyethylenglycol(14)isocetylether (lsoceteth-14), Polyethylenglycol(15)isocetylether (lsoceteth-15), Polyethylenglycol(16)isocetylether (lsoceteth-16), Polyethylenglycol(17)isocetylether (lsoceteth-17), Polyethylenglycol(18)isocetylether (lsoceteth-18), Polyethylenglycol(19)isocetylether (lsoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (lsolaureth-12).
Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)-stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)-isostearat, Polyethylenglycol(20)isostearat, Polyethylenglycol(21)isostearat, Polyethylenglycol(22)isostearat, Polyethylenglycol(23)isostearat, Polyethylenglycol(24)-isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet-werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)-sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Es ist dem Fachmanne natürlich bekannt, dass anspruchsvolle kosmetische Zusammensetzungen zumeist nicht ohne die üblichen Hilfs- und Zusatzstoffe denkbar sind. Die erfindungsgemäßen kosmetischen Zubereitungen können daher kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Parfüme, Pflanzenextrakte, Perlglanzagenzien, Mittel zum Verhindern des Schäumens, Farbstoffe, Pigmente mit färbender Wirkung, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Unreine Haut und Akne sind häufig miteinander verknüpft und ein Grundproblem dieser Hautzustände liegt in einem gestörten Zusammenspiel von Talgproduktion und Sekretion der Schweißdrüsen. Wirkstoffe, die gegen ein übermäßige Talgproduktion wirken, haben in der Regel einen günstigen Einfluss auf die unreine Haut bzw. Akne. Erfindungsgemäß können als günstige Wirkstoffe gegen übermäßige Talgproduktion, unreine Haut und/oder Akne alle für kosmetische und/oder dermatologische Anwendungen geeigneten oder gebräuchlichen Wirkstoffe gegen übermäßige Talgproduktion, unreine Haut und/oder Akne verwendet werden.

Als vorteilhaft werden die im Folgenden aufgeführten Wirkstoffe angesehen, die allein und/oder in Kombination eingesetzt werden können:
- Salicylsäure
- α-Hydroxycarbonsäuren
- Monoglycerin-monocarbonsäure-monoester, Diglycerin-monocarbonsäuremonoester, Triglycerin-monocarbonsäure-monoester, insbesondere partiell neutralisierte Ester von Monoglyceriden und/oder Diglyceriden mit Citronensäure
- Retinol
- Cycldxtrine.

Es kann erfindungsgemäß vorteilhaft sein, den Zubereitungen gemäß der Erfindung weitere gegen Sebum wirksame Agentien zuzufügen, beispielsweise gewählt aus der Gruppe Distärkephosphat, Natrium-Maisstärke-Octylensuccinat, Silica-Dimethyl-Silylat, Hydroxypropyl-Distärkephosphate, Tapioca-Dextrin, Aluminium-Stärke-Octylensuccinat, Acrylat-Copolymer, Magnesiumcarbonat, Maisstärke, Aluminium-Stärke-Octylensuccinate, Kaolin, Nylon (z.B. Nylon 6/12), Bentonit, Natrium-Silicoaluminat, Bornitrid, Silica, Glimmer + Titandioxid.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, □-Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glykosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glykosylrutin, Ferulasäure, Furfurylideriglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄), Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige-Konzentrationen aus dem Bereich-von 0,001- 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die' erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoäsäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfatoverbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Verwendung einer Kombination der erfindungsgemäß verwendeten Wirkstoffkombinationen mit mindestens einem UVB-Filter als Antioxidans bzw. die Verwendung einer Kombination der erfindungsgemäß verwendeten Wirkstoffkombinationen mit mindestens einem UVB-Filter als Antioxidans in einer kosmetischen oder dermatologischen Zubereitung.

Es kann auch von Vorteil sein, die erfindungsgemäß verwendeten Wirkstoffkombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'tert.Butylphenyl)-3-(4'-methoxyphenyl)propan-1,3-dion und um 1-Phenyl-3-(4'isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung ist auch die Verwendung einer Kombination von erfindungsgemäß verwendeten Wirkstoffkombinationen mit mindestens einem UVA-Filter als Antioxidans bzw. die Verwendung einer Kombination der erfindungsgemäßen Wirkstoffkombinationen mit mindestens einem UVA-Filter als Antioxidans in einer kosmetischen oder dermatologischen Zubereitung.

Gegenstand der Erfindung ist auch die Verwendung einer Kombination aus erfindungsgemäß verwendeten Wirkstoffkombinationen mit mindestens einem UVA-Filter und mindestens einem UVB-Filter als Antioxidans bzw. die Verwendung einer Kombination aus Wirkstoffkombinationen mit mindestens einem UVA-Filter und mindestens einem UVB-Filter als Antioxidans in einer kosmetischen oder dermatologischen Zubereitung.

Kosmetische und dermatologische Zubereitungen mit einem wirksamen Gehalt an erfindungsgemäß verwendeten Wirkstoffkombinationen können auch anorganische Pigmente enthalten, die üblicherweise in der Kosmetik zum Schütze der Haut vor UV-Strahlen verwendet werden. Dabei handelt es sich um Oxide des Titans, Zinks, Zirkoniums, Siliciums, Mangans, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Die folgenden **Beispiele** sollen die Erfindung erläutern, aber nicht einschränken. Die Zahlenangaben beziehen sich auf Gew.-%, sofern nichts anderes angegeben ist.

### W/O-Emulsionen

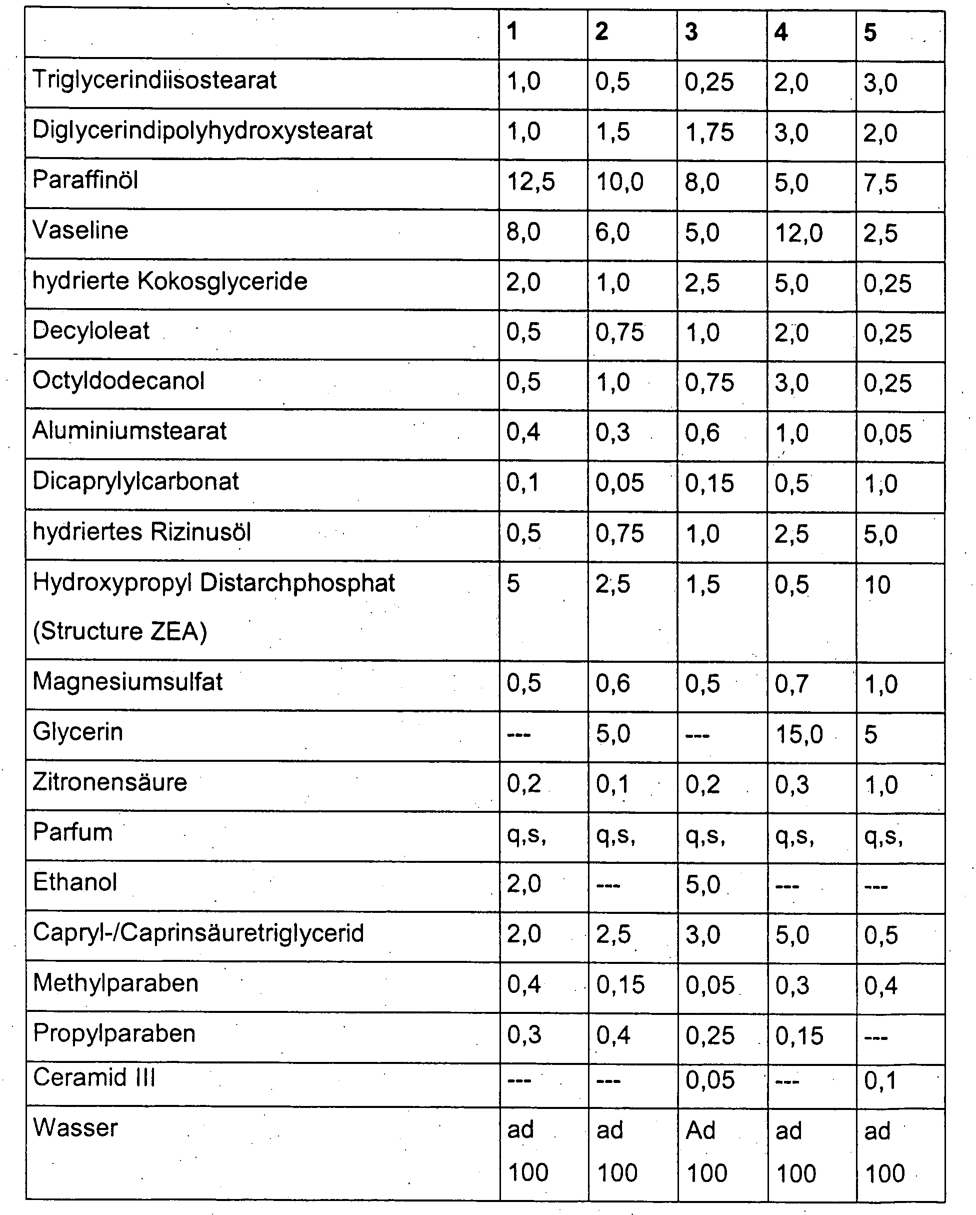

### W/O-Emulsionen

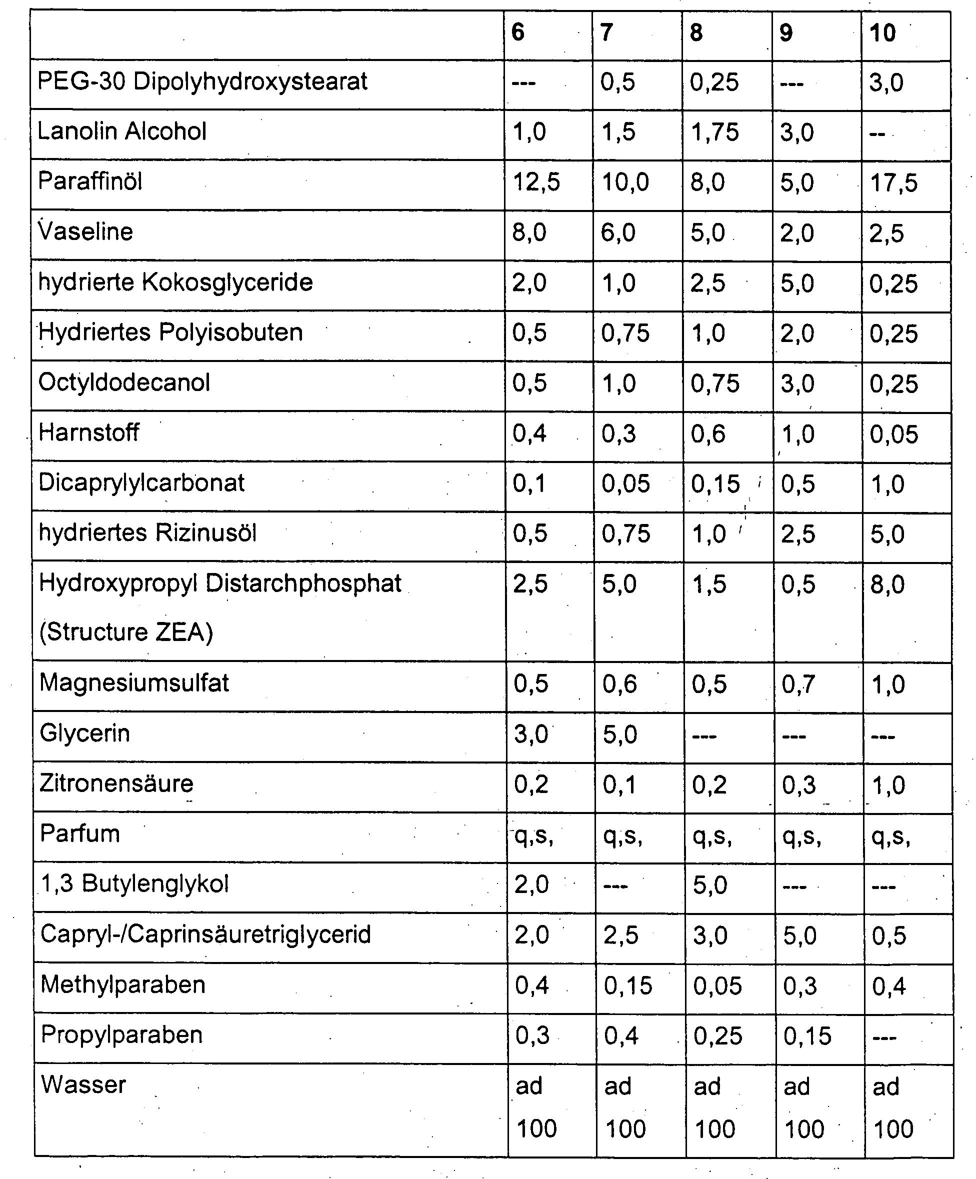

### W/S-Emulsion

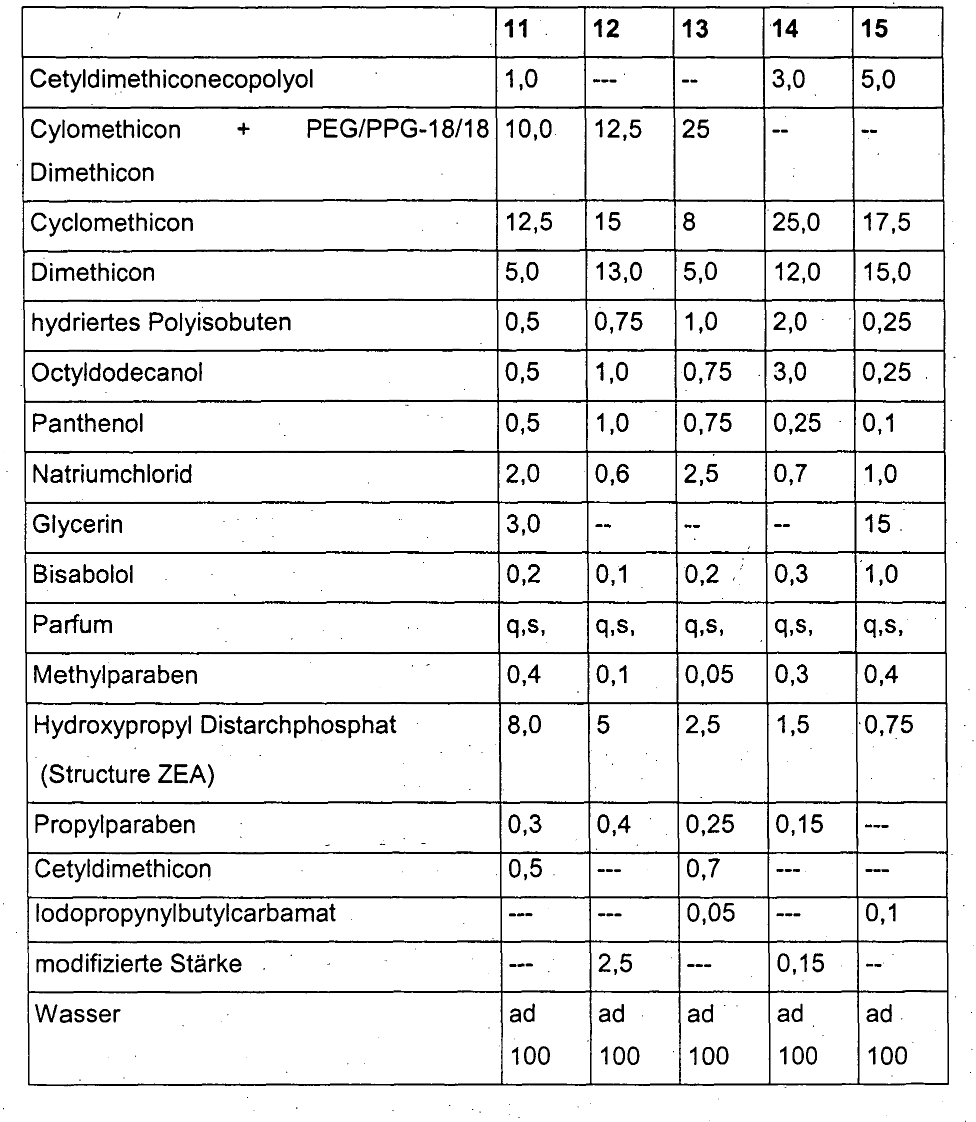

### W/S-Emulsionen

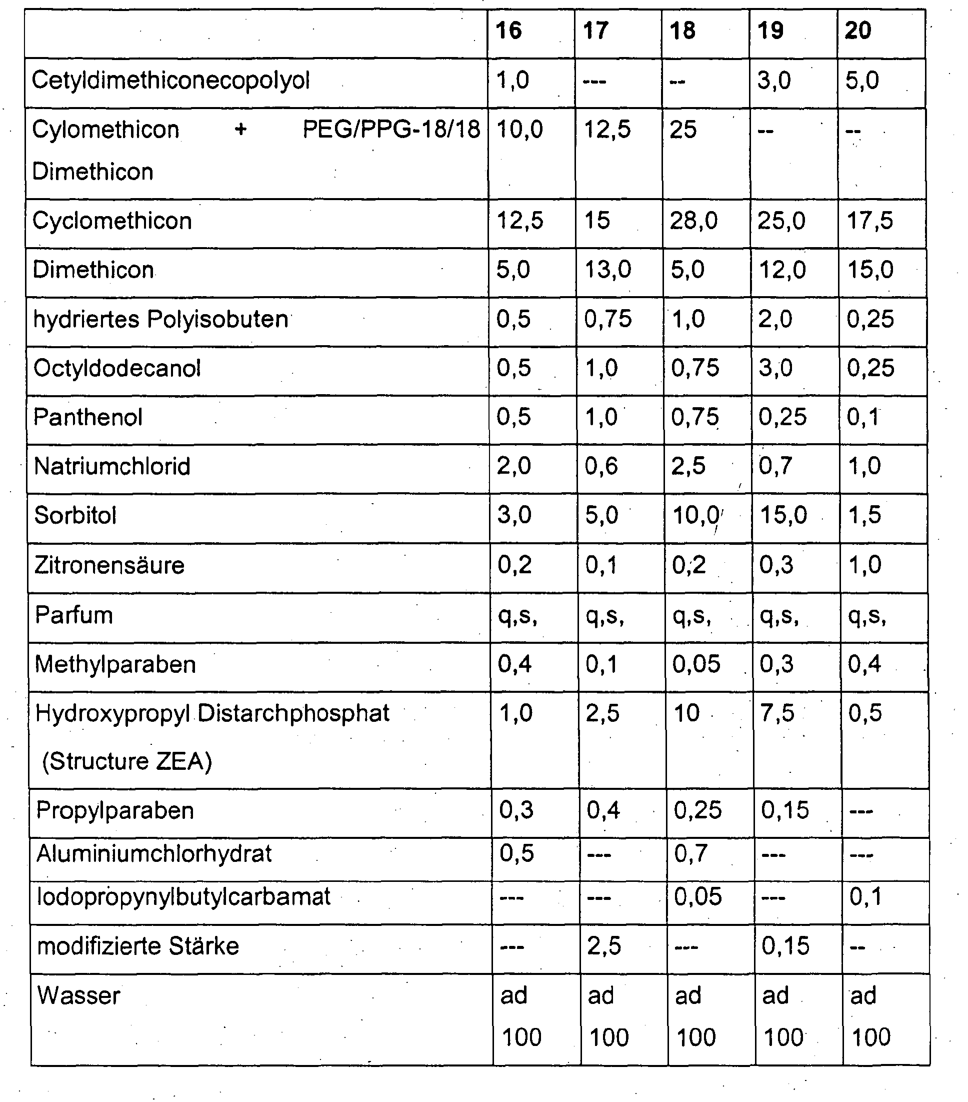

### Öl-in-Wasser-Emulsion

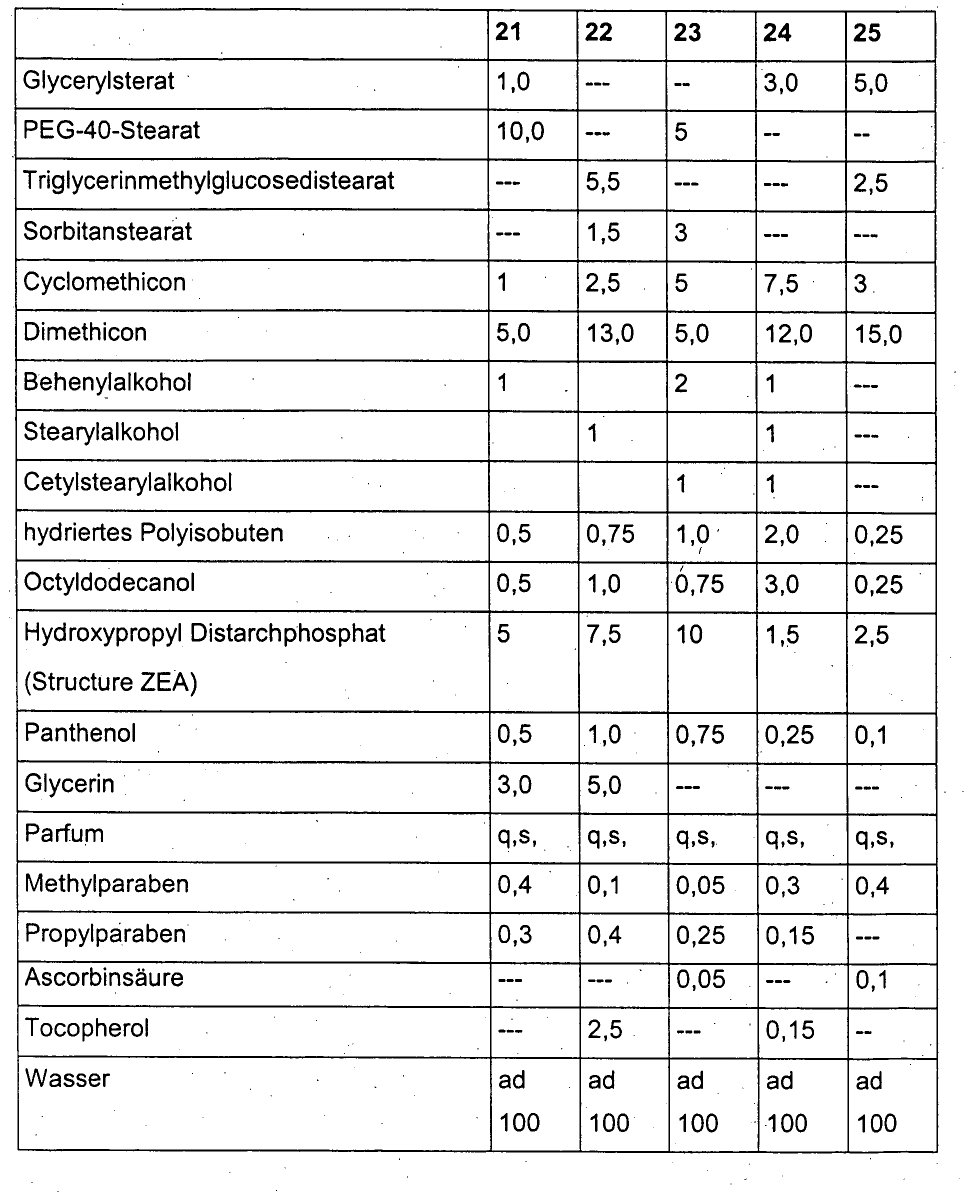

### Öl-in-Wasser-Emulsionen

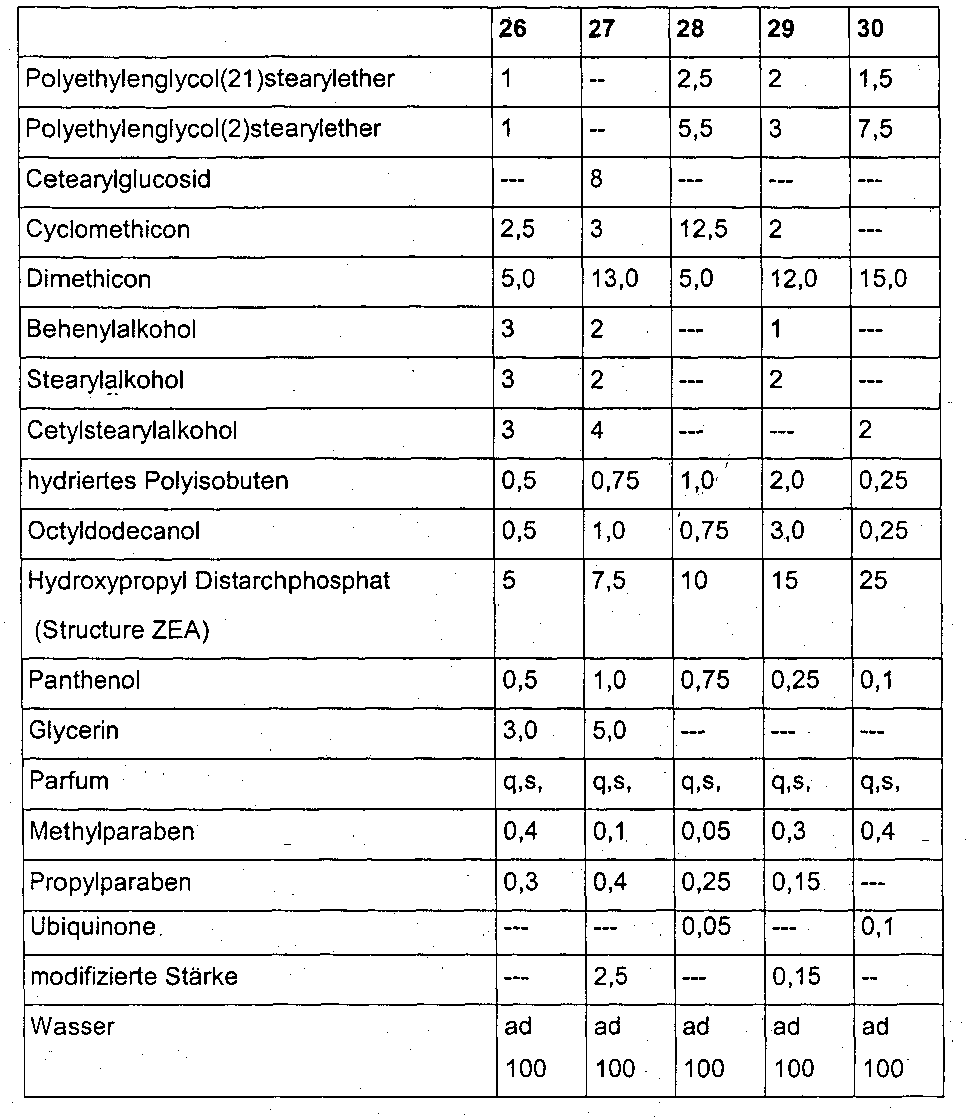

### Öl-in-Wasser-Emulsionen

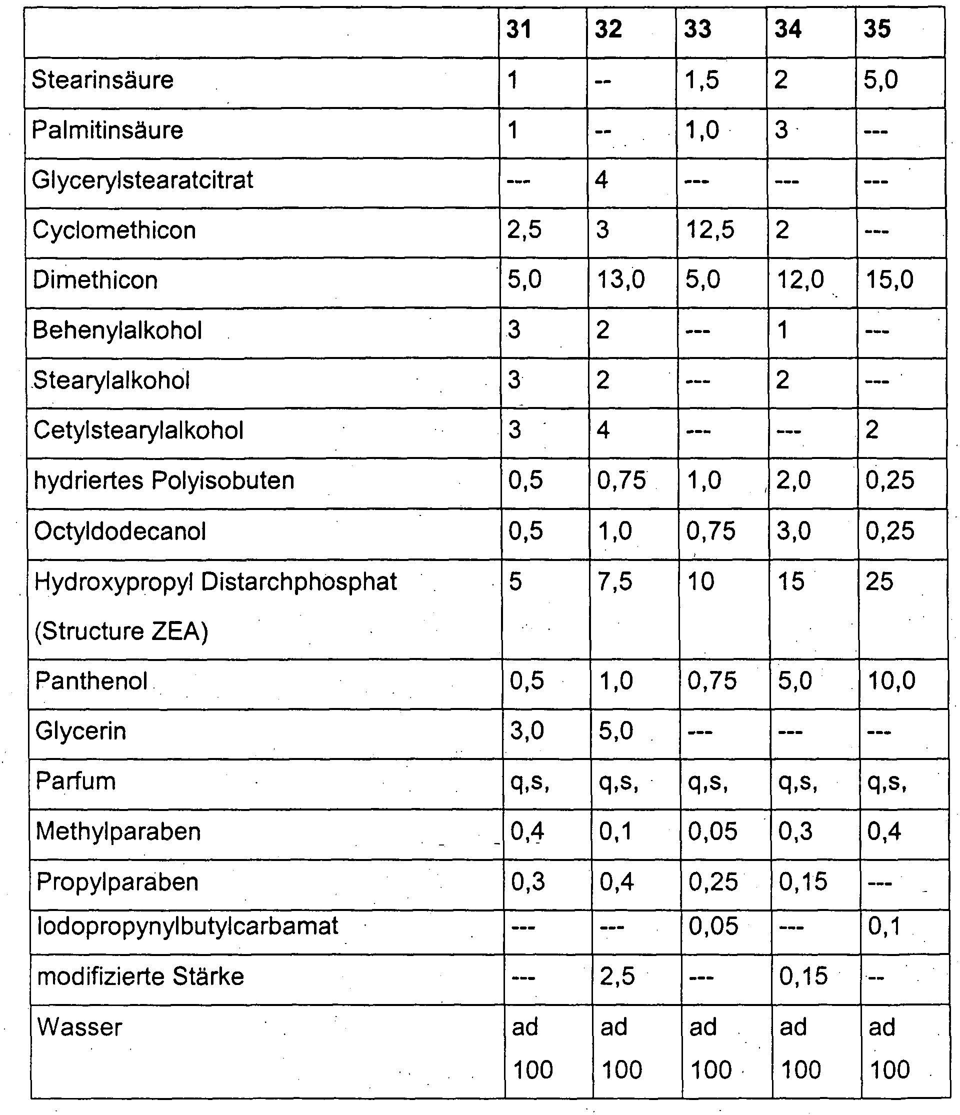

### Öl-in-Wasser-Emulsionen

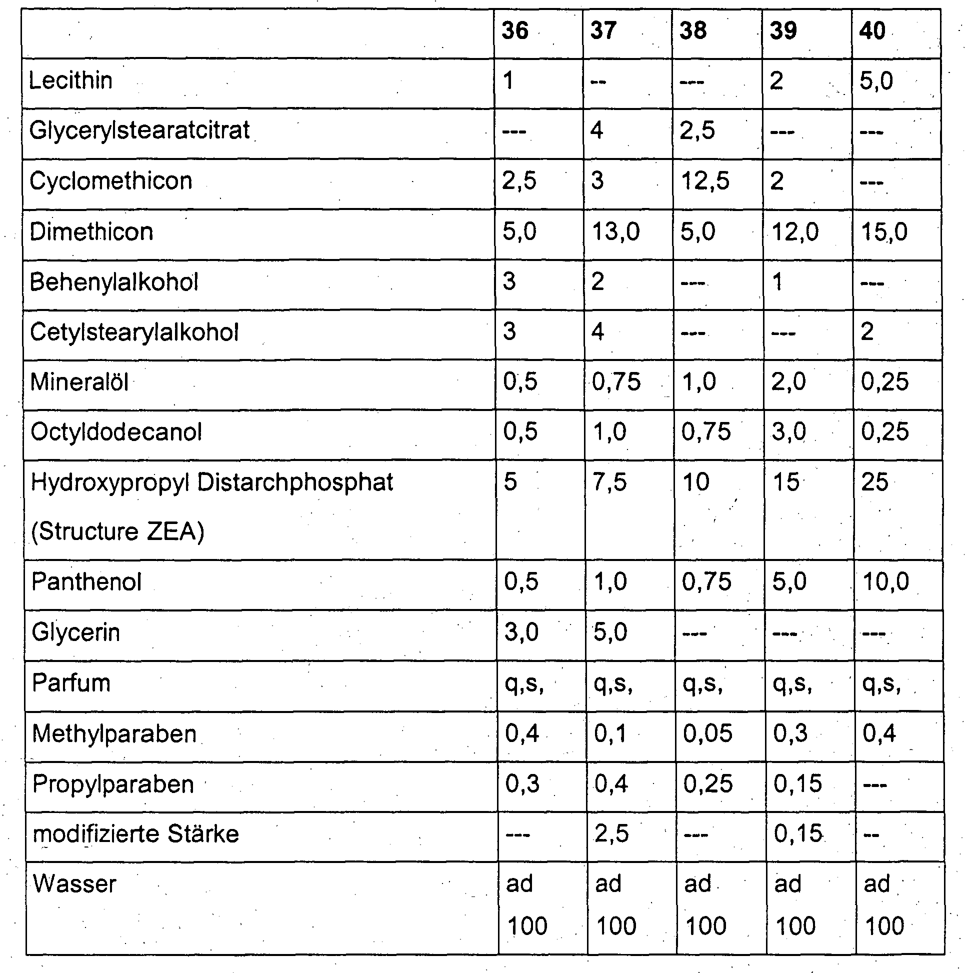

## Patentansprüche

1. Verwendung von Hydroxypropylstärkephosphat (Struktur ZEA), welches als Hydrokolloid vorliegt, allein oder in Kombination mit weiteren Hydrokolloiden zur verbesserten Verfügbarkeit von in kosmetischen und/oder dermatologischen Zubereitungen enthaltenen Wirkstoffen.

2. Verwendung von Hydroxypropylstärkephosphat (Struktur ZEA) nach Anspruch 1, allein oder in Kombination mit weiteren Hydrokolloiden zur verbesserten Verfügbarkeit von in kosmetischen und/oder dermatologischen Zubereitungen enthaltenen Wirkstoffen, insbesondere Wirkstoffen zur Prophylaxe, Pflege und Behandlung von fettiger Haut, unreiner Haut und Akne in unterschiedlich starken Ausprägungen.

3. Verwendung von Hydroxypropylstärkephosphat (Struktur ZEA) nach Anspruch 1, allein oder in Kombination mit weiteren Hydrokolloiden zur verbesserten Verfügbarkeit von in kosmetischen und/oder dermatologischen Zubereitungen enthaltenen Wirkstoffen, insbesondere Wirkstoffen zur Prophylaxe, Pflege und Behandlung von trockener und/oder empfindlicher Haut.

4. Verwendung von Hydroxypropylstärkephosphat (Struktur ZEA) nach Anspruch 1, allein oder in Kombination mit weiteren Hydrokolloiden zur verbesserten Verfügbarkeit von in kosmetischen und/oder dermatologischen Zubereitungen enthaltenen Wirkstoffen, insbesondere Wirkstoffen zur Prophylaxe, Pflege und Behandlung von Hautzuständen, die durch Falten und/oder andere äußere und innere Zeichen der Hautalterung gekennzeichnet sind.

5. Verwendung von Hydroxypropylstärkephosphat (Struktur ZEA) nach Anspruch 1, allein oder in Kombination mit weiteren Hydrokolloiden natürlichen oder synthetischen Ursprungs, wie z.B. PEG-Distearat, PEG 150 Distearat und anderen zur verbesserten Verfügbarkeit von in kosmetischen und/oder dermatologischen Zubereitungen enthaltenen Wirkstoffen.

6. Kosmetische und oder dermatologische Zubereitungen nach Anspruch 1 **gekennzeichnet durch** einen Gehalt von Hydroxypropylstärkephosphat (Struktur ZEA) von 0,1 bis 10 Gew.-%, bevorzugt von 0,3 bis 8,0 Gew.-%, insbesondere bevorzugt von 0,5 bis 5,0 Gew.-%.
